# EUROPÄISCHE PATENTSCHRIFT

(11) **EP 2 314 801 B1**
(45) Veröffentlichungstag und Bekanntmachung des Hinweises auf die Patenterteilung: **29.10.2014**
(21) Anmeldenummer: 10008369.0
(22) Anmeldetag: 11.08.2010
(51) Int. Cl.: E05B 1/00, A61L 2/10

(54) **Griff für Flügelelement**
Handle for leaf element
Poignée pour élément de battant

(30) Priorität: 20.10.2009 DE 102009050080
(43) Veröffentlichungstag der Anmeldung: 27.04.2011
(73) Patentinhaber: Dorma GmbH + Co. KG, 58256 Ennepetal (DE)
(72) Erfinder: Brieseck, Bernd, 58840 Plettenberg (DE)

(56) Entgegenhaltungen:
- FR-A1- 2 922 932
- JP-A- 2003 307 049
- JP-U- S5 589 754
- JP-U- S61 177 645
- US-A1- 2005 267 233
- US-B1- 7 175 807

## Beschreibung

Die vorliegende Erfindung betrifft einen Griff für ein Flügelelement mit einer UV-Strahlungsquelle zum Desinfizieren des Griffs, sowie ein Flügelelement mit dem Griff.

Die Flügelelemente von Türen oder Fenstern besitzen Beschläge, die zur Bedienung der Flügelelemente manuell bedienbare Elemente aufweisen. Die manuell bedienbaren Elemente können beispielsweise Türdrücker, Türknäufe oder Griffelemente, wie Fenstergriffe, sein. Im Folgenden wird ein manuell bedienbares Element nur kurz "Griff" genannt, soll aber nicht auf die Bedeutung eines Griffs im engeren Sinne beschränkt sein, sondern umfasst alle denkbaren Elemente, an die manuell zum Öffnen angegriffen werden kann. Derartige Griffe werden in Abhängigkeit des Einsatzorts und -zwecks von einer Vielzahl von Personen bedient.

In den letzten Jahren haben spezifische Probleme bezogen auf Infektionen zugenommen, die durch Kontakt mit manuell bedienbaren Elementen auftreten können. Ist das Flügelelement beispielsweise ein Türblatt einer Tür in einem öffentlichen Gebäude, einer öffentlichen Toilette, einem Krankenhaus, einem Hotel, einem Altenheim oder einer Arztpraxis, können Infektionsprobleme verstärkt auftreten. Die Gefahr einer Infektion kann verringert werden, wenn die Gefahr einer Infektion durch Berührung manuell bedienbarer Beschläge mit bakteriziden Mitteln verringert wird.

Es wurden verschiedene Methoden und Einrichtungen vorgeschlagen, um Beschläge an Flügelelementen zu desinfizieren.

Die JP 2003307049 offenbart eine Sterilisationsvorrichtung für Türgriffe, um Bakterien, die sich auf der Oberfläche des Türgriffs befinden, zu sterilisieren. Hierzu wird eine Sterilisationslampe genutzt, von der aus eine Strahlung auf den Türgriff ausgeübt wird. Eine Bestrahlungssteuerungsvorrichtung steuert die Strahlung der Sterilisationslampe auf den Türgriff. Weiterhin werden bei der Sterilisationsvorrichtung Abschirmungen genutzt, welche eine Reflektion der Lichtstrahlen aufnehmen, damit die von dem Türgriff reflektierten Lichtstrahlen nicht in die Augen eines Benutzers des Türgriffs gelangen. Die Bestrahlungssteuerungsvorrichtung aktiviert die Sterilisationslampe bei jeder Nutzung des Türgriffs durch den Benutzer, wodurch der Energieaufwand für die Strahlung der Sterilisationslampe entsprechend ist.

Aus der DE 103 05 142 A1 ist ein Türgriff mit einer Oberfläche bekannt, die eine keimtötende Wirkung besitzt. Die Oberfläche ist mit Substanzen behandelt, die eine bakterizide Wirkung besitzen. Diese Substanzen haben jedoch den Nachteil, dass sie entweder relativ toxisch sind oder ihre keimtötende Wirkung zu gering ist. Bei einem regelmäßigen Gebrauch des manuell bedienbaren Elements ist ein Abrieb der Substanzen festzustellen, wodurch die keimtötende Wirkung nachlässt. Ferner wird vorgeschlagen, das manuell bedienbare Element mit einem Metallkörper zu umhüllen, der nach Abrieb der keimtötenden Oberfläche ausgetauscht werden kann. Dies hat jedoch einen erheblichen Wartungsaufwand zur Folge.

Alternativ wurde vorgeschlagen, ein Flügelelement mit einer UV-Strahlungsquelle zu versehen, die zur bakteriziden UV-Bestrahlung des bedienbaren Elements ausgebildet ist. UV-Strahlen sind sehr gut geeignet, Bakterien, Viren, Pilze etc. sehr schnell abzutöten, ohne dass der Einsatz von Chemikalien nötig wäre.

Allerdings bestrahlt eine UV-Strahlungsquelle immer nur einen Teilbereich eines Griffs. Um einen größeren Bereich zu erreichen, muss eine Mehrzahl von UV-Lampen vorgesehen sein, was den Montageaufwand und die Anfälligkeit erhöht, sowie zu komplexen Anordnungen führt.

Aufgabe der Erfindung ist, die Probleme des genannten Standes der Technik zu überwinden. Insbesondere soll ein Flügelelement mit Griff und mit integrierter UV-Strahlungsquelle zum Desinfizieren des Griffs vorgeschlagen werden, wobei zumindest nahezu die vollständige Oberfläche des Griffs, die bei seiner Bedienung berührt wird, abhängig von der tatsächlichen Nutzung optimal und zuverlässig durch die Desinfektionsvorrichtung desinfizierbar ist.

Diese Aufgabe wird mit sämtlichen Merkmalen des Anspruches 1 gelöst. Vorteilhafte Weiterbildungen der Erfindung sind in den abhängigen Ansprüchen 2 bis 10 angegeben.

Erfindungswesentlich ist, dass die UV-Strahlungsquelle den Griff zumindest teilweise umgebend ausgebildet ist. Mit dem erfindungsgemäßen Griff wird das aus dem Stand der Technik bekannte Problem gelöst, dass nur ein Teilbereich des Griffs durch eine in ein Flügelelement integrierte Desinfektionsvorrichtung desinfiziert wird, während ein anderer Teilbereich stets durch eine Abschattung beispielsweise des Griffs selbst außerhalb eines Wirkbereichs einer UV-Strahlungsquelle liegt. Dabei hat die Erfindung den Vorteil, dass der Griff durch eine einzelne UV-Strahlungsquelle vollständig desinfiziert werden kann. Dies verringert Montage- und Wartungsaufwand gegenüber komplexeren Lösungen erheblich.

Erfindungsgemäß ist die UV-Strahlungsquelle in einem Gehäuse angeordnet, das einen Mantel aufweist. Dabei ist der Griff zumindest teilweise in einem Innenraum des Gehäuses angeordnet wobei der Innenraum durch den Mantel begrenzt ist.

Das Gehäuse ist geeignet, die UV-Strahlungsquelle gegenüber äußeren Einwirkungen zu schützen. Des Weiteren wirkt das Gehäuse nach außen gegenüber UV-Strahlen abschirmend.

Der Griff kann als Drehgriff ausgebildet sein. Unter Drehgriff wird im Rahmen der Erfindung ein Griff verstanden, der nicht durch Herunterdrücken einer Klinke bedienbar ist, sondern durch ein einfaches Drehen. Derartige Drehgriffe sind vor allem für das Schließen von Türblättern geeignet, die lediglich geringen Anforderungen hinsichtlich mechanischer Widerstandskraft, Einbruchsicherheit, Schallschutz und ähnlichem genügen müssen und deswegen in einer leichtgewichtigen Bauweise ausgeführt sind. Dies können beispielsweise Toilettentüren sein. Wegen der genannten geringen Anforderungen können derartige Drehgriffe mit geringen Abmessungen ausgebildet sein, da zum Öffnen einer leichtgewichtigen Tür nur geringe Kräfte angewendet werden müssen.

Der Griff kann zylinderförmig, insbesondere kreiszylinderförmig, ausgebildet sein. Dies hat den Vorteil, dass der Griff mit geringen Abmessungen platzsparend ausgebildet sein kann, wodurch der Mantel, das Gehäuse und die UV-Strahlungsquelle ebenfalls mit geringen Abmessungen ausgebildet werden kann. Unter zylinderförmig ist im Rahmen der Erfindung auch ein zumindest annähernd scheibenförmiger Griff zu verstehen, wie er bei Toilettentüren üblich ist. Der scheibenförmige Griff kann zur besseren Bedienbarkeit ein Greifelement, wie eine wulstartige Erhebung, aufweisen. Der Mantel ist erfindungsgemäß hohlzylinderförmig ausgebildet. Ein derartiger Mantel ist besonders gut geeignet, einen zylinderförmigen Griff platzsparend aufzunehmen und ist leicht herzustellen. Erfindungsgemäß ist zumindest eine Steuerung vorgesehen, die geeignet ist, eine Aktivität der UV-Strahlungsquelle zu steuern.
In Verbindung mit der Steuerung sind vorteilhafterweise Parameter, wie UV-Strahlungsdauer, -intensität und -weilenlänge so einstellbar, dass ein Desinfizieren des Griffs vorbestimmt erfolgen kann.
Nach einem Aspekt der Erfindung sind eine Energieversorgungseinheit, wie ein Stromnetzteil, und elektrische Leitungen zur Stromversorgung der Steuerung, der UV-Strahlungsquelle und möglicher weiterer Komponenten vorgesehen.
Vorzugsweise ist ein Sensor vorgesehen, der die Anwesenheit einer Person in der Nähe des Flügelelements detektiert. Dies hat den Vorteil, dass die UV-Strahlungsquelle nicht aktiviert wird und/oder eine Aktivität der UVStrahlungsquelle unterbrochen wird, wenn sich eine Person in der Nähe des Griffs befindet. Somit wird eine Verletzungsgefahr durch UV-Strahlung ausgeschlossen.
Es kann ein Mechanismus vorgesehen sein, der unmittelbar nach dem Zufallen der Tür oder währenddessen einen Desinfektionsvorgang in Gang setzt. Dadurch findet unmittelbar nach jeder Bedienung des Flügelelements ein Desinfizieren des Griffs statt. Dies hat den Vorteil, dass der Griff unmittelbar nach jeder Berührung desinfiziert wird und sich Keime daher nicht erst vermehren können. Außerdem entfernt sich im Normalfall die Person, die die Tür bedient hat. nach einem Durchschreiten von der Tür, so dass die Desinfektion durch UV-Bestrahlung erfolgen kann, ohne dass eine Person dadurch geschädigt werden kann, bzw. ohne dass ein Desinfektionsschritt wegen der Nähe der Person unterbleiben muss.
Alternativ ist es auch als vorteilhaft vorstellbar, dass die Steuerung so eingestellt ist, dass ein Desinfizieren automatisch in regelmäßigen zeitlichen Abständen durchgeführt wird.
Erfindungsgemäß ist eine Zähleinrichtung vorgesehen, die die Anzahl der Bedienungen des Griffs oder die Anzahl der Bedienungen des Flügelelements bestimmt. Die Steuerung aktiviert nach einer vorbestimmten Anzahl von Bedienungen ein Desinfizieren des Griffs mittels der V-Strahlungsquelle. Die Anzahl der Bedienungen ist vorteilhafterweise manuell leicht einstellbar und kann vorzugsweise in Abhängigkeit von diversen Parametern, wie der Tageszeit oder einer Infektionsgefahrstufe, programmiert werden.
Nach einer vorteilhaften Ausführungsform weist der Mantel eine Mantelinnenfläche auf und ist die UV-Strahlungsquelle mit der Mantelinnenfläche verbunden. Dadurch ist die UV-Strahlungsquelle mechanisch gestützt.

Vorteilhafterweise ist zwischen der UV-Strahlungsquelle und der Mantelinnenfläche zumindest teilbereichsweise ein Reflektor vorgesehen, der geeignet ist, eine von der UV-Strahlungsquelle erzeugte UV-Strahlung zu reflektieren. Der Reflektor ermöglicht, dass UV-Strahlung, die von der UV-Strahlungsquelle zunächst in einer vom Griff abgewandten Richtung emittiert wird, zum Griff hin umgelenkt werden kann. Somit erhöht sich ein Verhältnis von ausgesandter UV-Strahlungsmenge zu einer UV-Strahlungsmenge, die auf den Griff trifft. Dadurch kann beispielsweise eine UV-Strahlungsquelle geringerer Leistung eingesetzt werden, als ohne den Einsatz eines Reflektors nötig wäre, um eine selbe Desinfektionsrate zu erzielen.

Des Weiteren kann der Mantel durch den Einsatz des Reflektors vorteilhafterweise aus einem Material gefertigt sein, das keine hohe UV-Beständigkeit aufweist, denn der Mantel wird durch den Reflektor gegen UV-Strahlen geschützt. Dies ermöglicht den Einsatz eines kostengünstigen Materials für den Mantel, wie eines herkömmlichen, nicht UV-beständigen Kunststoffs.

Vorteilhafterweise kann eine die UV-Strahlungsquelle zum Innenraum hin abdeckende Abdeckung vorgesehen sein, die für eine von der UV-Strahlungsquelle erzeugte UV-Strahlung durchlässig ist. Die Abdeckung schützt die UV-Strahlungsquelle vor mechanischen Beschädigungen, beispielsweise durch Berührung einer den Griff bedienenden Person. Andererseits gewährt die Abdeckung einen Schutz für eine den Griff bedienende Person. So schützt die Abdeckung vor Verbrennungen durch Berührung mit der UV-Strahlungsquelle, die unmittelbar nach Durchführung eines Desinfektionsschritts eine hohe Temperatur erreicht haben kann.

Vorteilhafterweise enthält die Abdeckung Quarzglas, das eine hohe UV-Durchlässigkeit besitzt und gleichzeitig eine schlechte Wärmeleitfähigkeit aufweist. Dadurch erwärmt sich die Abdeckung durch die UV-Strahlungsquelle kaum.

Vorteilhafterweise ist die Abdeckung so ausgebildet, dass sie den Reflektor und/oder die Mantelinnenfläche zumindest teilweise abdeckt. Das hat den Vorteil, dass zwischen der Abdeckung und dem Reflektor und/der Mantelinnenfläche keine Stufen entstehen. Dadurch ist das Gehäuse leicht zu reinigen, und eine Verletzungsgefahr durch Stufen wird vermieden.

Nach einer vorteilhaften Variante weist eine Mantelaußenfläche des Mantels eine desinfizierende Oberfläche auf. Die Mantelaußenfläche wird nicht von der UV-Strahlung der UV-Strahlungsquelle getroffen. Daher ist es von Vorteil, wenn die Mantelaußenfläche selbst desinfizierend ist. Da die Mantelaußenfläche bei einer Bedienung des Griffs nicht berührt werden muss, sondern höchstens selten, beispielsweise versehentlich, von einer den Griff bedienenden Person mit einer Hand berührt wird, sind im Einsatz des Griffs nur wenige Keime auf der Mantelaußenfläche zu erwarten. Daher ist eine Oberfläche, die eine nur langsam wirkende Desinfektion bewirkt, ausreichend. Da die Mantelaußenfläche einer nur sehr geringen mechanischen Belastung durch Berührung von Personen ausgesetzt ist, besteht nicht die Gefahr, dass die desinfizierende Oberfläche verschlissen und abgerieben wird und somit die desinfizierende Wirkung verloren geht.

Unter desinfizierend soll im Rahmen der Erfindung jede Wirkung verstanden werden, die eine keimtötende, bakterizide, viruzide, fungizide oder ähnliche Wirkung ist, wobei die genaue Wirkungsweise oder Intensität der Wirkung für die Erfindung unerheblich ist.

Das Gehäuse kann eine Stirnwand aufweisen, die geeignet ist, an einem Flügelelement angeordnet zu sein. Somit lässt sich das Gehäuse ohne großen Montageaufwand an einem Flügelelement anbringen. Die Stirnwand schützt darüber hinaus das Flügelelement, an dem das Gehäuse angebracht werden soll, vor UV-Strahlung.

Vorteilhafterweise weist die Stirnwand eine dem Innenraum zugewandte Stirnfläche auf, die eine desinfizierende Oberfläche besitzt. Auch die Stirnfläche kann eventuell bei einer manuellen Bedienung des Griffs von der bedienenden Person berührt werden. Daher können auch auf der Stirnfläche Keime vorliegen. Insbesondere, falls die Stirnfläche nicht vollständig von den UV-Strahlen erreicht wird, ist es daher vorteilhaft, wenn die Stirnfläche bereits selbst desinfizierend ist. Da die Stirnfläche einer nur sehr geringen mechanischen Belastung durch Berührung von Personen ausgesetzt ist, besteht nicht die Gefahr, dass die desinfizierende Oberfläche verschlissen und abgerieben wird und somit die desinfizierende Wirkung verloren geht.

Zumindest eine der desinfizierenden Oberflächen kann Kupfer und/oder eine desinfizierende Nanokompositoberfläche aufweisen. Kupfer oder Nanokompositoberflächen haben sich als besonders wirksam zur Desinfizierung erwiesen. Da die desinfizierenden Oberflächen selten berührt werden, sind dort nur wenige Keime vorhanden, so dass eine relativ langsame desinfizierende Wirkung zur Desinfizierung genügt.

Vorteilhafterweise ist die UV-Strahlungsquelle zur Erzeugung von UV-Strahlung mit Wellenlängen zwischen 100 und 380 nm, insbesondere zwischen 150 und 280 nm (UV-C-Strahlung), besonders bevorzugt zwischen 250 und 260 nm geeignet. Bei blauer UV-Strahlung zwischen 250 und 260 nm, insbesondere etwa 254 nm sind Abtötungsraten gegenüber Keimen besonders hoch.

Alternativ kann ein Wellenlängenbereich zwischen 315 und 380 nm, der mit UV-A bezeichnet wird, vorteilhaft zur Desinfizierung sein.

Gelöst wird die Aufgabe der Erfindung des Weiteren mit sämtlichen Merkmalen des Patentanspruchs 9. Vorteilhafte Aus- und Weiterbildungen des Flügelelements sind in dem abhängigen Anspruch 10 angegeben.

Weitere, die Erfindung verbessernde Maßnahmen werden nachstehend gemeinsam mit der Beschreibung eines bevorzugten Ausführungsbeispiels der Erfindung anhand der Figuren näher dargestellt.

Dabei zeigen:

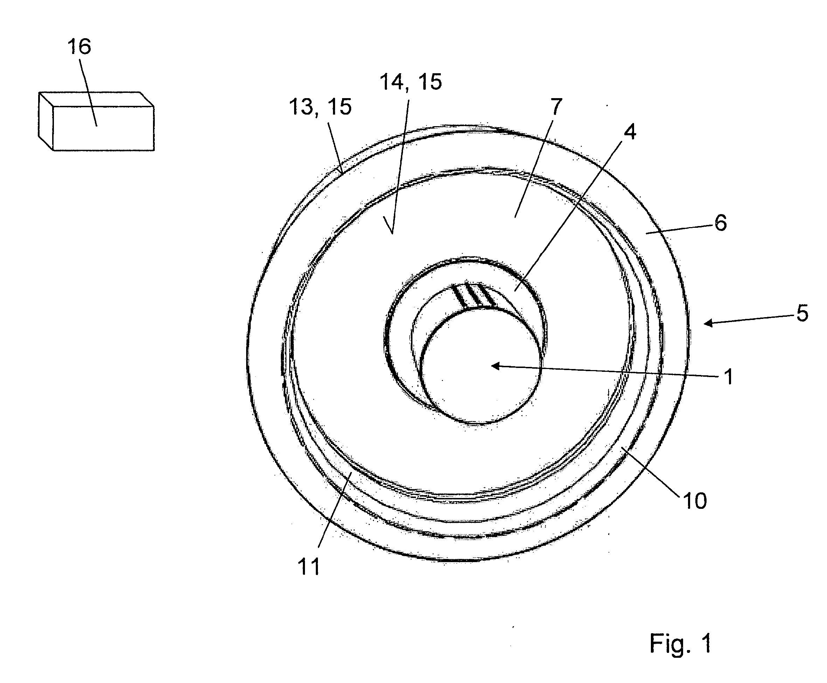

- Fig. 1:: eine perspektivische Gesamtansicht eines schematisch dargestellten erfindungsgemäßen Griffs im montierten Zustand,
- Fig. 2:: eine perspektivische Ansicht eines Griffs und getrennt davon eines Gehäuses mit ringförmiger UV-Strahlungsquelle,
- Fig. 3:: eine Ansicht eines Griffs und getrennt davon eine perspektivische Explosionsansicht eines Gehäuses mit ringförmiger UV-Strahlungsquelle und

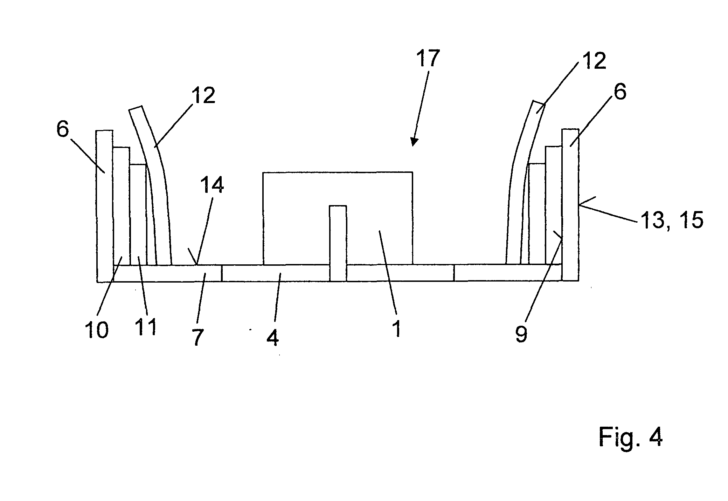

- Fig. 4:: eine Schnittansicht eines Griffs mit Gehäuse und ringförmiger Strahlungsquelle.

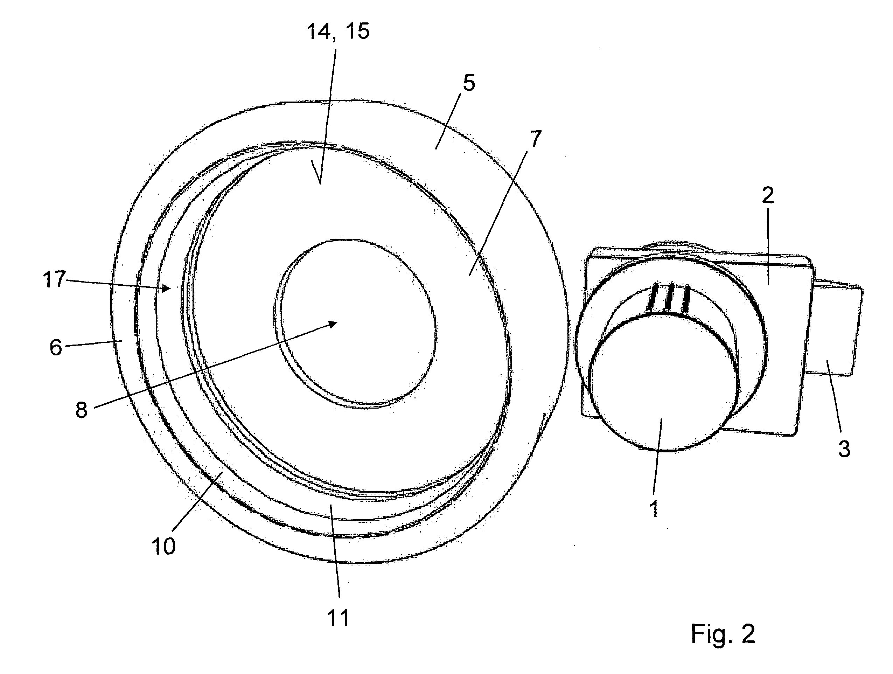

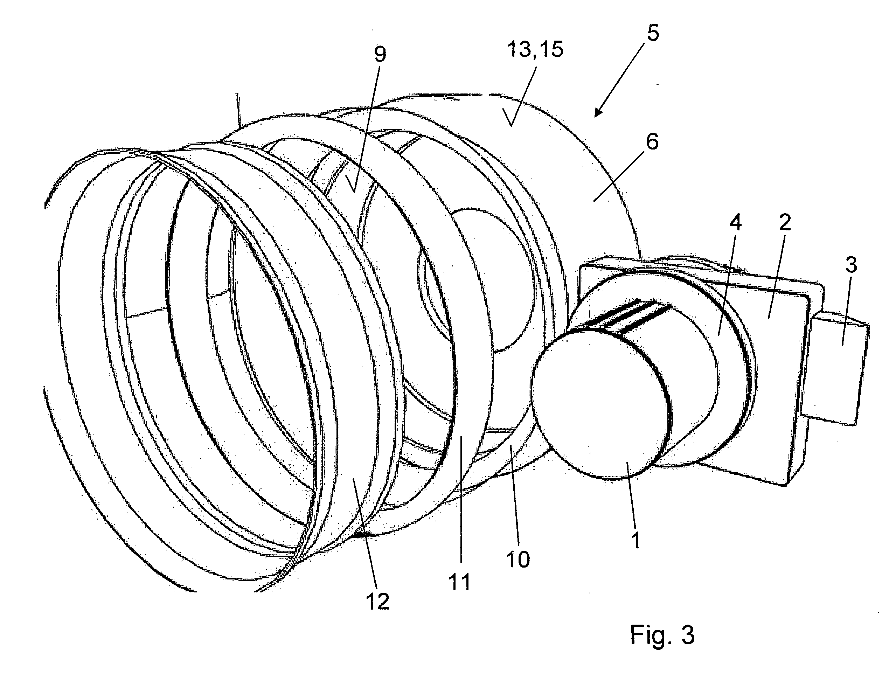

Ein erfindungsgemäßer Griff 1, der gemäß dem Ausführungsbeispiel als Toilettentürgriff 1 ausgebildet ist, ist an einem Flügelelement 2, das in diesem Beispiel eine Toilettentür 2 ist, die in Fig. 2 und 3 nur schematisch als Ausschnitt eines Türblatts 2 angedeutet ist, drehbar gelagert. Der Toilettentürgriff 1 ist als kreiszylinderförmiger Drehgriff 1 ausgebildet, d.h. lediglich durch ein Drehen des Griffs 1 kann ein Verschlussriegel 3 bewegt werden und dadurch die Toilettentür 2 verriegelt und entriegelt werden.

In diesem Ausführungsbeispiel besitzt der Drehgriff 1 einen ringförmigen Fuß 4. Um den Griff 1 ist ein Gehäuse 5 angeordnet, das einen hohlzylinderförmigen Mantel 6 und eine Stirnwand 7 umfasst. Die Stirnwand 7 weist eine kreisförmige Ausnehmung 8 auf, deren Durchmesser weitgehend dem Durchmesser des Fußes 4 entspricht, so dass die Stirnwand 7 zu einem Montieren des Gehäuses 5 an der Toilettentür 2 über den Drehgriff 1 mit dem Fuß 4 gepasst wird. Im Falle gleicher Wanddicken der Stirnwand 7 und des Fußes 4, wie in diesem Beispiel ergibt sich eine bündige Fläche aus Stirnwand 7 und Fuß 4. In einem montierten Zustand befindet sich der Griff 1 in einem vom Gehäuse 5 begrenzten Innenraum 17.

In diesem Ausführungsbeispiel ist auf einer Mantelinnenfläche 9 des Mantels 6 ein Reflektor 10 aufgebracht, der geeignet ist, UV-Strahlen zu reflektieren. Auf dem Reflektor 10 ist eine ringförmige UV-Strahlungsquelle 11 vorgesehen, die in diesem Beispiel eine UV-Lampe 11 in Ringform ist.

Der Mantel 6 ist in diesem Ausführungsbeispiel höher als der Drehgriff 1 ausgebildet, wodurch der Drehgriff 1 vollständig im Innenraum 17 des Mantels 6 und somit auch innerhalb des Gehäuses 5 angeordnet ist. Alternativ kann der Mantel 6 aber auch niedriger ausgebildet sein, so dass der Mantel 6 nur geringfügig höher als der Griff 1, gleichhoch oder sogar niedriger als der Griff 1 ausgebildet ist. Je nachdem wie von der UV-Lampe 11 ausgestrahlte UV-Strahlen fokussiert oder weit aufgefächert sind, kann in Kombination mit einer Breite der UV-Lampe 11 und/oder des Reflektors 10 eine für den jeweiligen Anwendungsfall gewünschte geometrische Ausbildung des Gehäuses 5 und des Griffs 1 vorgesehen sein. Sowohl die Breite der UV-Lampe 11 und des Reflektors 10 als auch die Höhe des Mantels 6 sind dabei in axialer Richtung des Gehäuses 5 definiert.

In einer Variante des Ausführungsbeispiels ist eine Abdeckung 12 aus UVdurchlässigem Quarzglas vorgesehen, die hohlzylinderförmig ausgebildet ist und die ringförmige UV-Lampe 11, den Reflektor 10 und die Mantelinnenfläche 9 des Mantels 6 abdeckt.

Eine Mantelaußenfläche 13 des Mantels 6 und eine Stirnfläche 14 der Stirnwand 7 sind als Kupferoberfläche 15 ausgebildet. Die Mantelaußenfläche 13 wird von der UV-Lampe 11 ausgesandtem UV-Licht nicht erreicht und kann somit auch nicht dadurch desinfiziert werden. Da Kupfer keimtötend wirkt, werden auf der Mantelaußenfläche 13 vorhandene Keime, Bakterien, Pilze und ähnliches zwar langsamer als durch UV-Licht, aber wirkungsvoll abgetötet. Die Stirnfläche 14 liegt zwar innerhalb des Gehäuses 5, kann aber zumindest in Teilbereichen durch den Griff 1 abgeschattet sein oder anderweitig schlecht für UV-Strahlen erreichbar sein. Daher ist in diesem Beispiel auch die Stirnfläche 14 als Kupferoberfläche 15 ausgebildet. Alternativ sind andere selbst desinfizierende Oberflächen, wie selbst desinfizierende Nanokompositoberflächen denkbar.

Die UV-Lampe 11 des Beispiels sendet im Betrieb blaues UV-Licht mit einer Wellenlänge von etwa 254 nm aus, das somit sogenanntes UV-C-Licht darstellt, das bei dieser Wellenlänge besonders hohe Abtötungsraten erzeugt. Außer UV-C-Licht im Bereich von 100 bis 280 nm ist beispielsweise auch UV-A-Licht im Bereich von 315 bis 380 nm zum Desinfizieren geeignet. Des Weiteren ist selbstverständlich auch UV-B-Strahlung zwischen 280 und 315 nm geeignet.

Durch regelmäßiges Aktivieren der UV-Lampe 11 wird der Griff 1 jedesmal desinfiziert und eine Keimausbreitung und -vermehrung wirksam unterdrückt. In diesem Beispiel wird die UV-Lampe 11 in regelmäßigen zeitlichen Abständen, wie beispielsweise 10 min, für einige Sekunden aktiviert. Je nach Einsatzort, Tageszeit und weiteren Faktoren kann die UV-Lampe 11 auch mit kürzeren und längeren Zeitabständen oder mit einer kürzeren oder längeren Aktivierungsdauer eingesetzt werden.

Alternativ kann die UV-Lampe 11 aufgrund einer durch eine Zähleinrichtung (nicht dargestellt) bestimmte Anzahl von Türbedienungen oder anderweitige Kriterien mittels einer Steuerung (nicht dargestellt) aktiviert und deaktiviert werden.

Befindet sich eine Person in der Nähe des Griffs 1, detektiert dies ein Sensor 16, und die UV-Lampe 11 wird deaktiviert.

UV-Lampe 11, Sensor 16 und Steuerung sind mit einer nicht in den Figuren dargestellten Stromversorgung versehen.

Ein erfindungsgemäßer Griff 1 mit UV-Lampe 11 kann wie beschrieben auf einer oder auf beiden Seiten eines Flügelelements 2 vorgesehen sein. Die Erfindung ist nicht auf Drehgriffe 1 an Toilettentüren 2 beschränkt, sondern kann auf alle Griffe 1 an Flügelelementen 2 übertragen werden, es muss lediglich eine UV-Strahlungsquelle 11 zumindest teilweise einen Griff 1 umgeben.

Die UV-Strahlungsquelle 11 kann des Weiteren mehrstückig ausgebildet sein. Dies kann durch Anordnung einer Mehrzahl von UV-Lampen 11 in radialer und/oder axialer Richtung des Gehäuses 5 bewerkstelligt sein.

### Bezugszeichenliste

- 1: Griff, Toilettentürgriff, Drehgriff
- 2: Flügelelement, Türblatt, Toilettentür
- 3: Verschlussriegel
- 4: Fuß
- 5: Gehäuse
- 6: Mantel
- 7: Stirnwand
- 8: Ausnehmung
- 9: Mantelinnenfläche
- 10: Reflektor
- 11: UV-Strahlungsquelle, UV-Lampe
- 12: Abdeckung
- 13: Mantelaußenfläche
- 14: Stirnfläche
- 15: Desinfizierende Oberfläche, Kupferoberfläche, Nanokompositoberfläche
- 16: Sensor
- 17: Innenraum

## Patentansprüche

1. Anordnung mit einem Griff (1) für ein Flügelelement (2), insbesondere für ein Türblatt, einem Gehäuse (5) und einer UV-Strahlungsquelle (11) zum Desinfizieren des Griffs (1) wobei die UV-Strahlungsquelle (11) den Griff (1) ringförmig umgebend ausgebildet ist, wobei die UV-Strahlungsquelle (11) im Gehäuse (5) angeordnet ist, das einen Mantel (6) aufweist, wobei der Griff (1) zumindest teilweise in einem Innenraum (17) des Gehäuses (5) angeordnet ist, der durch den Mantel (6) begrenzt ist, wobei der Mantel (6) zylinderförmig ausgebildet ist, **dadurch gekennzeichnet, dass** die Anordnung eine Steuerung und eine Zähleinrichtung aufweist, und die UV-Strahlungsquelle (11) aufgrund einer durch die Zähleinrichtung bestimmten Anzahl von Türbedienungen mittels der Steuerung aktivierbar und deaktivierbar ist.

2. Anordnung mit einem Griff (1) nach Anspruch 1, **dadurch gekennzeichnet, dass** der Mantel (6) eine Mantelinnenfläche (9) aufweist und zwischen der UV-Strahlungsquelle (11) und der Mantelinnenfläche (9) zumindest teilbereichsweise ein Reflektor (10) vorgesehen ist, der geeignet ist, eine von der UV-Strahlungsquelle (11) erzeugte UV-Strahlung zu reflektieren.

3. Anordnung mit einem Griff (1) nach einem oder mehreren der vorhergehenden Ansprüche, **dadurch gekennzeichnet, dass** eine die UV-Strahlungsquelle (11) abdeckende Abdeckung (12) vorgesehen ist, die für eine von der UV-Strahlungsquelle (11) erzeugte UV-Strahlung durchlässig ist.

4. Anordnung mit einem Griff (1) nach einem oder mehreren der Ansprüche 1 bis 3, **dadurch gekennzeichnet, dass** der Mantel (6) eine Mantelaußenfläche (13) aufweist und die Mantelaußenfläche (13) eine desinfizierende Oberfläche (15) aufweist.

5. Anordnung mit einem Griff (1) nach einem oder mehreren der Ansprüche 1 bis 4, **dadurch gekennzeichnet, dass** das Gehäuse (5) eine Stirnwand (7) aufweist, die geeignet ist, an einem Flügelelement (2) angeordnet zu sein.

6. Anordnung mit einem Griff (1) nach Anspruch 5, **dadurch gekennzeichnet, dass** die Stirnwand (7) eine dem Innenraum (17) zugewandte Stirnfläche (14) aufweist, die eine desinfizierende Oberfläche (15) besitzt.

7. Anordnung mit einem Griff (1) nach einem oder mehreren der Ansprüche 4 bis 6, **dadurch gekennzeichnet, dass** zumindest eine desinfizierende Oberfläche (15) Kupfer und/oder eine desinfizierende Nanokompositoberfläche aufweist.

8. Anordnung mit einem Griff (1) nach einem oder mehreren der vorhergehenden Ansprüche, **dadurch gekennzeichnet, dass** die UV-Strahlungsquelle (11) geeignet ist, UV-Strahlung im Bereich von UV-A-Strahlung und/oder UV-C-Strahlung zu erzeugen.

9. Flügelelement (2), insbesondere Türblatt oder Fensterblatt, mit einer Anordnung mit einem Griff (1) nach einem oder mehreren der Ansprüche 1 bis 8.

10. Flügelelement (2) nach Anspruch 9, **dadurch gekennzeichnet, dass** das Flügelelement (2) einen Sensor (16) aufweist, der geeignet ist, eine Person in einem Wirkbereich der UV-Strahlungsquelle (11) zu detektieren.

## Claims

1. An arrangement including a handle (1) for a leaf element (2), in particular for a door leaf, a housing (5) and a UV-radiation source (11) for disinfecting the handle (1), wherein the UV-radiation source (11) is configured to surround the handle (1) in a ring-shape manner, wherein the UV-radiation source (11) is disposed in the housing (5), which has a cladding (6), wherein the handle (1) is disposed, at least partially, in an inner space (17) of the housing (5), which space is delimited by the cladding (6), wherein the cladding (6) is configured to be cylindrically-shaped, **characterized in that** the arrangement features a control unit and a counting device, and **in that** the UV-radiation source (11), based on a certain number of door manipulations determined by the counting device, can be activated and deactivated by means of the control unit.

2. The arrangement including a handle (1) according to claim 1, **characterized in that** the cladding (6) features a cladding inner surface (9), and **in that** a reflector (10) is provided at least in partial areas between the UV-radiation source (11) and the cladding inner surface (9), which reflector is suitable for reflecting a UV-radiation generated by the UV-radiation source (11).

3. The arrangement including a handle (1) according to one or more of the preceding claims, **characterized in that** a cover (12) covering the UV-radiation source (11) is provided, which is transmissible for a UV-radiation generated by the UV-radiation source (11).

4. The arrangement including a handle (1) according to one or more of the claims 1 to 3, **characterized in that** the cladding (6) features a cladding outer surface (13) and **in that** the cladding outer surface (13) features a disinfecting surface (15).

5. The arrangement including a handle (1) according to one or more of the claims 1 to 4, **characterized in that** the housing (5) features a frontal wall (7), which is suitable to be disposed at a leaf element (2).

6. The arrangement including a handle (1) according to claim 5, **characterized in that** the frontal face (7) features a frontal surface (14) oriented towards the inner space (17) and having a disinfecting surface (15).

7. The arrangement including a handle (1) according to one or more of the claims 4 to 6, **characterized in that** at least one disinfecting surface (15) features copper and/or a disinfecting nanocomposite surface.

8. The arrangement including a handle (1) according to one or more of the preceding claims, **characterized in that** the UV-radiation source (11) is suitable for generating UV-radiation in the range of UV-A radiation and/or UV-C radiation.

9. A leaf element (2), in particular a door leaf or a window leaf including an arrangement with a handle (1) according to one or more of the claims 1 to 8.

10. The leaf element (2) according to claim 9, **characterized in that** the leaf element (2) features a sensor (16) which is suitable to detect a person in an operative area of the UV-radiation source (11).

## Revendications

1. Agencement avec une poignée (1) pour un élément à vantail (2) tout particulièrement pour un vantail de porte, avec un boîtier (5) et une source de rayonnement UV (11) pour désinfecter la poignée (1), la source de rayonnement UV (11) étant agencée en forme d'anneau et de façon à entourer la poignée (1), la source de rayonnement UV (11) étant agencée dans le boîtier (5), qui présente un gainage (6), la poignée (1), au moins partiellement, étant agencée dans un espace intérieur (17) du boîtier (5), lequel espace est délimité par l'intermédiaire du gainage (6), le gainage (6) étant aménagé de façon cylindrique, **caractérisé en ce que** l'agencement présente une commande et un dispositif de comptage, et **en ce que** la source de rayonnement UV (11) peut être activée et désactivée au moyen de la commande sur la base d'un nombre de manipulations de la porte, nombre déterminé par l'intermédiaire du dispositif de comptage.

2. Agencement avec une poignée (1) selon la revendication 1, **caractérisé en ce que** le gainage (6) présente une surface intérieure de gainage (9) et **en ce qu'**un réflecteur est prévu au moins en certaines parties entre la source de rayonnement UV (11) et la surface intérieure de gainage (9), lequel réflecteur est susceptible de réfléchir un rayonnement UV produit par l'intermédiaire de la source de rayonnement UV (11).

3. Agencement avec une poignée (1) selon l'une ou plusieurs des revendications précédentes, **caractérisé en ce qu'**un recouvrement (12) couvrant la source de rayonnement UV (11) est prévu lequel est transmissible à un rayonnement UV produit par l'intermédiaire de la source de rayonnement UV (11).

4. Agencement avec une poignée (1) selon l'une ou plusieurs des revendications 1 à 3, **caractérisé en ce que** le gainage (6) présente une surface extérieure de gainage (13) et **en ce que** la surface extérieure de gainage (13) présente une surface désinfectante (15).

5. Agencement avec une poignée (1) selon l'une ou plusieurs des revendications 1 à 4, **caractérisé en ce que** le boîtier (5) présente une paroi frontale (7) qui est susceptible d'être agencée sur un élément de vantail (2).

6. Agencement avec une poignée (1) selon la revendication 5, **caractérisé en ce que** la paroi frontale (7) présente une surface frontale (14) orientée vers l'espace intérieur (17) laquelle surface frontale présente une surface désinfectante (15).

7. Agencement avec une poignée (1) selon l'une ou plusieurs des revendications 4 à 6, **caractérisé en ce qu'**au moins une surface désinfectante (15) présente du cuivre et/ou une surface nanocomposite désinfectante.

8. Agencement avec une poignée (1) selon l'une ou plusieurs des revendications précédentes, **caractérisé en ce que** la source de rayonnement UV (11) est susceptible de produire du rayonnement UV dans le domaine du rayonnement UV-A et/ou du rayonnement UV-C.

9. Elément à vantail (2), en particulier un vantail de porte ou vantail de fenêtre comprenant un agencement avec une poignée (1) selon l'une ou plusieurs des revendications 1 à 8.

10. Elément à vantail (2) selon la revendication 9, **caractérisé en ce que** l'élément à vantail (2) présente un capteur (16) qui est susceptible de détecter une personne dans une zone d'active de la source de rayonnement UV (11).
